# EUROPEAN PATENT APPLICATION

(11) **EP 3 275 477 A1**
(43) Date of publication of application: **31.01.2018**
(21) Application number: 16768906.6
(22) Date of filing: 24.03.2016
(51) Int. Cl.: A61M 5/162, A61J 1/06, A61J 1/20, A61J 3/00

(54) **MEDICAL RESIN-MADE HOLLOW NEEDLE, OUTER CYLINDER PROVIDED WITH PUNCTURE PART, AND PRE-FILLED SYRINGE**

(30) Priority: 26.03.2015 JP 2015065359
(71) Applicant: Terumo Kabushiki Kaisha, Tokyo 151-0072 (JP)
(72) Inventor: IIBUCHI Ruriko, Ashigarakami-gun Kanagawa 259-0151 (JP); FUKE Shigeaki, Ashigarakami-gun Kanagawa 259-0151 (JP); ARINOBE Manabu, Ashigarakami-gun Kanagawa 259-0151 (JP)
(74) Representative: Casalonga
(86) International application number: PCT/JP2016/059469
(87) International publication number: WO 2016/153003

(57) **Abstract**

A medical resin-made hollow needle 1 is provided with a distal-end part 3 having a puncturing end part 2, a large-diameter part 4 having a larger diameter than the distal-end part 3, a tapered part 6 provided between the large-diameter part and the distal-end part, and a hub part 5. The distal-end part is provided with a tapered puncturing end part 2 and a hole 7 communicating the inside and outside of the hollow needle and being provided a predetermined length toward the proximal end relative to a distal end 2a and a predetermined length toward the distal end relative to the tapered part. The distal-end part 3 has an overall length L of 7-14 mm and a maximum outside diameter R of 1.5-3.0 mm, the tapered part 6 has a length O of 0.3-4.7 mm and a taper angle (taper spread angle) of 10-50 degrees, and the large-diameter part 4 has a length N of 4 mm or greater and a distal-end outside diameter Q which is 0.6-3.0 mm larger than the maximum outside diameter R of the distal-end part 3.

## Description

### Technical Field

The present invention relates to a medical resin-made hollow needle for medical use, an outer cylinder with puncture portion, and a pre-filled syringe using the same. Specifically, the present invention relates to a medical resin-made hollow needle that is capable of performing connection to a vial container with puncture, and connection to a mixed injection port or a discharging port of an infusion solution container with the puncture, a medical device set using the medical resin-made hollow needle, an outer cylinder with puncture portion, and a pre-filled syringe using the outer cylinder.

### Background Art

As the medical resin-made hollow needle, various needles are suggested.

As the medical resin-made hollow needle, for example, a needle disclosed in JP 2005-323622 A (Patent Literature 1) can be exemplified. A resin-made needle member disclosed in Patent Literature 1 is used when injecting a medical solution into a drip infusion tube that is used in a medical site. The resin-made needle member disclosed in Patent Literature 1 includes a needle section and a mounting section. The needle section includes a forward section, an intermediate section, and a proximal end section. The needle section is formed integrally with the mounting section through the proximal end section. An outer diameter of the forward section is set to 1.0 mm or less, and an inner diameter thereof is set to less than 0.8. An outer diameter of a lowest portion of the intermediate section is set to 2.0 mm or less, and an inner diameter thereof is set to less than 0.8. A curvature R is formed between the intermediate section and the proximal end section.

### Citation List

### Patent Literature

Patent Literature 1: JP 2005-323622 A

### Summary of Invention

### Technical Problem

The needle member disclosed in Patent Literature 1 is effective when injecting a medical solution into the drip infusion tube.

The present inventors have made a thorough investigation, and they found that it is preferable, from the viewpoints of working efficiency, prevention of erroneous administration of drugs, and the like, to perform a series of the following operations, that is, an operation of puncturing a vial container, an operation of suctioning a drug in the vial container, a connecting operation to a mixed injection port or a discharging port of the infusion solution container as is, and an operation of injecting a medical solution into the infusion solution container.

However, in the needle member disclosed in Patent Literature 1, an insertion possible portion is long, and when suctioning the entirety of a solution from the vial, it is necessary to perform adjustment while observing a hole position. Accordingly, a needle may fall out or time is taken to suction the solution. This situation is also true of a metallic needle that is used in the related art, and thus a medical resin-made hollow needle having a short length is also suggested. On the other hand, with regard to injection of the medical solution into the infusion solution container, in a case where a needle having a length suitable for suctioning of a drug from the vial punctures a port of the infusion solution container, a connection state does not become stable because a puncture portion is thin and short. Accordingly, the medical resin-made hollow needle has a problem that it is difficult to stably puncture both the vial and the infusion solution container by using the same needle .

An object of the invention is to provide a medical resin-made hollow needle capable of performing connection to a vial container with puncture, and stable connection to a mixed injection port or a discharging port of an infusion solution container with the puncture, a medical device set using the medical resin-made hollow needle, an outer cylinder with puncture portion, and a pre-filled syringe using the outer cylinder.

### Solution to Problem

To accomplish the object, there is provided the following configuration.

A medical resin-made hollow needle, comprising:
a distal end section including a puncture end portion at a distal end thereof;
a large-diameter section having a diameter greater than a diameter of the distal end section, and a predetermined length;
a tapered section that is provided between the large-diameter section and the distal end section, and has a diameter that increases toward a proximal end thereof; and
a hub section that is provided in a proximal end of the hollow needle, and is attachable to a Luer-taper nozzle of a medical device,
wherein the distal end section includes a hole that communicates with an inside and an outside of the resin-made hollow needle,
wherein the distal end section has a total length in a range of 7 to 14 mm, and a maximum outer diameter in a range of 1.5 to 3.0 mm,
wherein the tapered section has a length in a range of 0.3 to 4.7 mm, and a taper angle in a range of 10° to 50°,
wherein the large-diameter section has a length in a range of 4 to 12 mm, and a distal end outer diameter that is greater than the maximum outer diameter of the distal end section by a range of 0.6 to 3.0 mm,
wherein the distal end section is configured to pierce an elastic container-sealing member that seals an opening of a vial container, and the distal end section and the tapered section are configured to pierce an elastic port-sealing member that seals an opening of a port of an infusion solution container, and
wherein the large-diameter section is configured to enter the elastic port-sealing member, and a state of the large-diameter section being entering into the elastic port-sealing member is configured to be maintained due to a pressure applied from the elastic port-sealing member.

In addition, to accomplish the object, there is provided the following configuration.

A medical device set, including:
the medical resin-made hollow needle; and
a medical device,
wherein the medical device is a pre-filled syringe that comprises an outer cylinder that includes an outer cylinder main body having a cylindrical shape and the Luer-taper nozzle provided at a distal end portion of the outer cylinder main body and including a distal end opening, a gasket that is slidably accommodated in the outer cylinder, a medical liquid that is filled in the outer cylinder, a sealing member that seals the distal end opening of the outer cylinder, and a plunger that is attached or attachable to a proximal end of the gasket.

In addition, to accomplish the object, there is provided the following configuration.

An outer cylinder with puncture portion, comprising:
an outer cylinder main body having a cylindrical shape; and
a hollow puncture portion that is provided in a distal end portion of the outer cylinder main body,
wherein the puncture portion comprises a distal end section that includes a puncture end portion at a distal end thereof, a large-diameter section having a diameter greater than a diameter of the distal end section and having a predetermined length, and a short tapered section that is provided between the large-diameter section and the distal end section,
wherein the distal end section includes a hole that communicates with an inside and an outside of the puncture portion, and the puncture end portion having a tapered shape,
wherein the distal end section has a total length in a range of 7 to 14 mm, and a maximum outer diameter in a range of 1.5 to 3.0 mm,
wherein the tapered section has a length in a range 0.3 to 4.7 mm, and a taper angle in a range of 10° to 50°,
wherein the large-diameter section has a length in a range 4 to 12 mm, and a distal end outer diameter that is greater than the maximum outer diameter of the distal end section by a range of 0.6 to 3.0 mm,
wherein the distal end section is configured to pierce an elastic container-sealing member of a vial container, and the distal end section and the tapered section are configured to pierce an elastic port-sealing member of a port of an infusion solution container, and
wherein the large-diameter section is configured to enter the elastic port-sealing member, and a state of the large-diameter section being entering into the elastic port-sealing member is configured to be maintained due to a pressure applied from the elastic port-sealing member.

In addition, to accomplish the object, there is provided the following configuration.

A pre-filled syringe, comprising:
the outer cylinder with puncture portion;
a gasket that is slidably accommodated in the outer cylinder;
a medical liquid that is filled in the outer cylinder; and
a plunger that is attached or attachable to a proximal end of the gasket.

### Brief Description of Drawings

Fig. 1 is a front view of a medical resin-made hollow needle of an example of the invention.
Fig. 2 is a bottom view of the medical resin-made hollow needle illustrated in Fig. 1.
Fig. 3 is a rear view of the medical resin-made hollow needle illustrated in Fig. 1.
Fig. 4 is a right side view of the medical resin-made hollow needle illustrated in Fig. 1.
Fig. 5 is a left side view of the medical resin-made hollow needle illustrated in Fig. 1.
Fig. 6 is a cross-sectional view taken along line A-A in Fig. 1.
Fig. 7 is a perspective view of the medical resin-made hollow needle illustrated in Fig. 1.
Fig. 8 is a view illustrating an operation of the medical resin-made hollow needle of the invention.
Fig. 9 is a view illustrating an operation of the medical resin-made hollow needle of the invention.
Fig. 10 is a view illustrating an operation of the medical resin-made hollow needle of the invention.
Fig. 11 is a view illustrating an operation of the medical resin-made hollow needle of the invention.
Fig. 12 is a front view of a medical resin-made hollow needle of another example of the invention.
Fig. 13 is a view illustrating an operation of the medical resin-made hollow needle of the example illustrated in Fig. 12.
Fig. 14 is a front view of a medical resin-made hollow needle of still another example of the invention.
Fig. 15 is a longitudinal cross-sectional view of the medical resin-made hollow needle illustrated in Fig. 14.
Fig. 16 is a front view of a medical device set according to an example of the invention.
Fig. 17 is a front view of an outer cylinder with puncture portion of an example of the invention.
Fig. 18 is a cross-sectional view taken along line B-B in Fig. 17.
Fig. 19 is an enlarged view of a distal end section of the outer cylinder with puncture portion illustrated in Fig. 18.
Fig. 20 is a front view of a pre-filled syringe that uses the outer cylinder with puncture portion illustrated in Fig. 17 to Fig. 19.
Fig. 21 is a view illustrating a distal end section of the pre-filled syringe illustrated in Fig. 20.

### Description of Embodiments

Description will be given of a medical resin-made hollow needle of the invention with reference to examples illustrated in the drawings.

A medical resin-made hollow needle 1 of the invention includes a distal end section 3 including a puncture end portion 2, a large-diameter section 4 that has a diameter greater than that of the distal end section 3 and a predetermined length, a short tapered section 6 that is provided between the large-diameter section 4 and the distal end section 3, and a hub section 5 that is provided in a proximal end and can be attached to a Luer-taper nozzle of a medical device.

The distal end section 3 includes lateral holes 7 (7a, 7b, and 7c) which are provided on a proximal end side in comparison to a distal end 2a and on a distal end side in comparison to the tapered section 6 by a predetermined length as holes which communicate with an inner side and an outer side of the medical resin-made hollow needle 1, and the puncture end portion 2 having a tapered shape. In addition, the distal end section 3 has a total length L of 7 to 14 mm and a maximum outer diameter R of 1.5 to 3.0 mm. The tapered section 6 has a length O of 0.3 to 4.7 mm and a taper angle (taper expansion angle) of 10° to 50°. The large-diameter section 4 has a length N of 4 mm or greater, and a distal end outer diameter Q greater than the maximum outer diameter R of the distal end section 3 by 0.6 to 3.0 mm.

In addition, the distal end section 3 can be inserted through an elastic container-sealing member 42 of a vial container 40. The distal end section 3 and the tapered section 6 can be inserted through an elastic port-sealing member 74 of a port 63 of an infusion solution container 70. The large-diameter section 4 can enter the inside of the elastic port-sealing member 74 of the port 63 of the infusion solution container 70, and a state, in which the large-diameter section 4 has entered the inside of the elastic port-sealing member 74, can be maintained due to a pressure applied from the elastic port-sealing member 74.

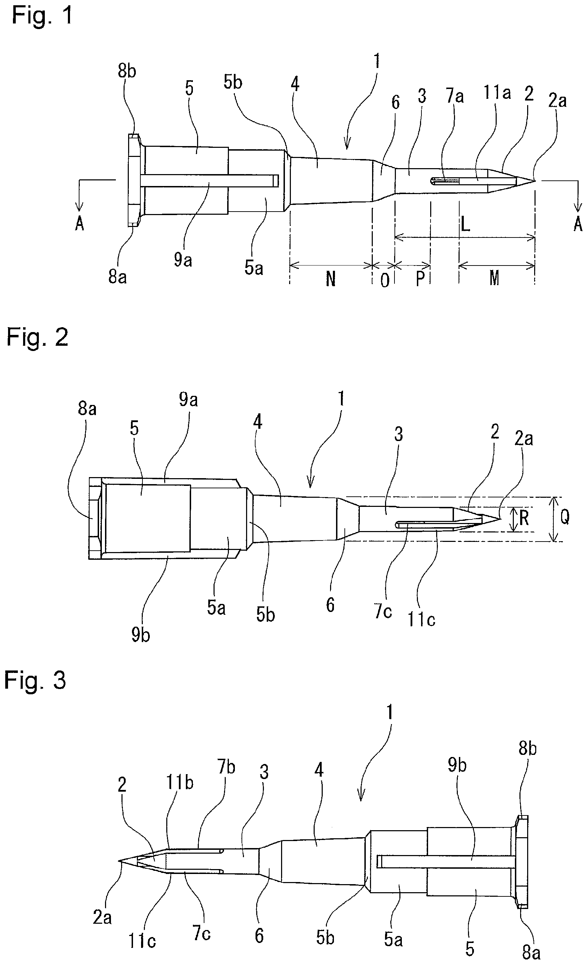

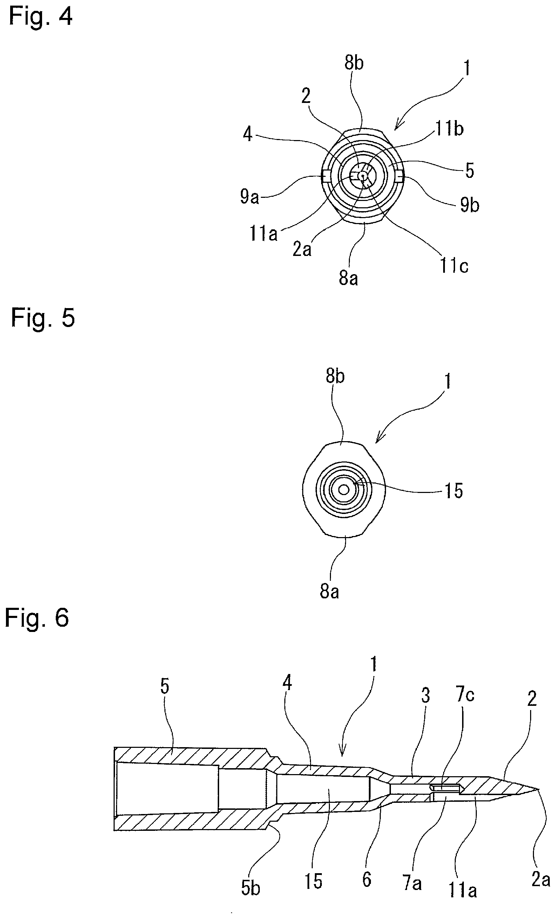

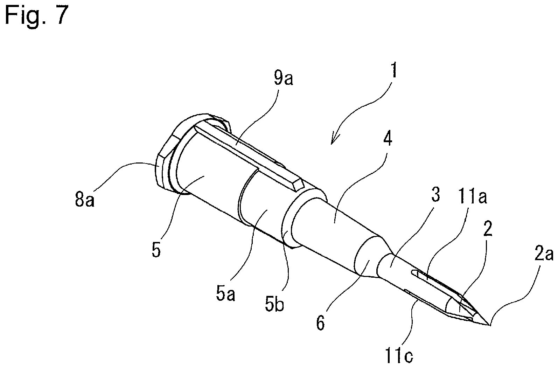

As illustrated in Fig. 1 to Fig. 7, the medical resin-made hollow needle 1 includes the distal end section 3 including the puncture end portion 2 that has a solid tapered shape from the distal end side, the tapered section 6, the large-diameter section 4, and the hub section 5. In addition, as illustrated in Fig. 6 and Fig. 7, the hollow needle 1 includes a lumen-like portion 15, of which a diameter decreases toward the distal end, at the inside thereof, and the lumen-like portion 15 communicates with the outside through the lateral holes 7 (7a, 7b, and 7c) . It is preferable that a diameter of the lumen-like portion 15 is 0.4 to 1.0 mm, and more preferably 0.6 to 0.8 mm.

As illustrated in Fig. 1 to Fig. 3, Fig. 6, and Fig. 7, the puncture end portion 2, which is formed in a distal end of the distal end section 3, is solid and has an acute taper angle (taper expansion angle) for forming a puncture tip from the distal end side. It is preferable that the tapered puncture end portion 2 has a conical shape having a total length of 1.0 to 4.7 mm and a taper angle of 12° to 45°. Particularly, it is preferable that the tapered puncture end portion 2 has a total length of 1.7 to 4.0 mm and a taper angle of 14° to 30°, and more preferably a total length of 2.1 to 4.0 mm and a taper angle of 14° to 25°. When the puncture end portion 2 is set to the tapered shape, and the lateral holes are provided on the proximal end side in comparison to the distal end 2a, it is easy for the distal end section 3 to be inserted through the elastic container-sealing member 42 of the vial container 40 and the elastic port-sealing member 74 of the port 63 of the infusion solution container 70, and coring is less likely to occur during insertion through the elastic sealing member.

The total length L of the distal end section 3 including the puncture end portion 2 is set to 7 to 14 mm, and the maximum outer diameter R of the distal end section 3 is set to 1.5 to 3.0 mm. Accordingly, the distal end section 3 including the puncture end portion 2 is sufficiently thin while maintaining strength, and has a length in an axial direction to a certain extent. As a result, it is easy to puncture the elastic sealing member 42 of the vial container 40 and the elastic sealing member 74 of the port 63 of the infusion solution container 70, and it is possible to confirm the puncture end portion 2 during puncturing. Furthermore, as the total length L is shorter, it is easier to maintain the strength of the distal end section 3. In addition, as the total length L is longer, it is easier to confirm the puncture end portion 2 during puncturing. To make the two situations be compatible with each other, it is preferable that the total length L is 8 to 12 mm, and more preferably 9 to 11 mm. In addition, it is preferable that the maximum outer diameter R of the distal end section 3 is 1.8 to 2.6 mm in order for the elastic sealing member 42 to be easily punctured with the distal end section 3 while maintaining the strength of the distal end section 3, and more preferably 2.0 to 2.4 mm.

In addition, the distal end section 3 excluding the puncture end portion 2 has a gently-sloping taper angle, and a proximal end thereof has the maximum diameter. Furthermore, the distal end section 3 excluding the puncture end portion 2 may not substantially have a taper angle . "May not substantially have taper" is a concept including "have a taper of approximately 0 to 5.5% (0 to approximately 3.15°)".

In addition, the medical resin-made hollow needle 1 includes the lateral holes 7 as a hole that communicates with an inner side and an outer side of the medical resin-made hollow needle 1. A distance P between a proximal end of the lateral holes 7 (7a, 7b, and 7c) and a proximal end of the distal end section 3 is set to 1.8 to 5.0 mm, and a length of the lateral holes 7 (7a, 7b, and 7c) in an axial direction is set to 1.0 to 2.5 mm. According to this, the proximal end of the lateral holes 7 (7a, 7b, and 7c) is spaced away from the distal end of the tapered section 6, and has a sufficient length in an axial direction. According to this, as to be described later, when the tapered section 6 reaches a ceiling surface of the elastic container-sealing member 42 during of puncturing of the hollow needle 1 into the elastic sealing member 42 of the vial container 40, it enters a state in which the lateral holes 7 (7a, 7b, and 7c) are opened to the vicinity of an inner surface of the elastic container-sealing member 42 and are not opened to the outside of the vial container 40.

Furthermore, it is preferable that the distance P between the proximal end of the lateral holes 7 (7a, 7b, and 7c) and the proximal end of the distal end section 3 is 2.0 to 4.0 mm, and more preferably 2.0 to 3.5 mm. According to this, when the tapered section 6 reaches the ceiling surface of the elastic container-sealing member 42, it enters a state in which the lateral holes 7 (7a, 7b, and 7c) are not reliably opened to the outside of the vial container 40. In addition, it is preferable that the length of the lateral holes 7 (7a, 7b, and 7c) in the axial direction is 1.5 to 2.5 mm. According to this, when the tapered section 6 reaches the ceiling surface of the elastic container-sealing member 42, it enters a state in which the lateral holes 7 (7a, 7b, and 7c) are reliably opened to the vicinity of the inner surface of the elastic container-sealing member 42. In addition, it is preferable that a distance M between a distal end of the lateral holes 7 (7a, 7b, and 7c) and the distal end 2a of the puncture end portion 2 is 3.0 to 7.5 mm, and more preferably 4.5 to 6.4 mm.

In addition, the medical resin-made hollow needle 1 of this example includes a plurality of, specifically, three lateral holes 7a, 7b, and 7c, and the respective lateral holes 7a, 7b, and 7c are formed at an equal angle with respect to a central axis of the hollow needle 1 as illustrated in Fig. 4 and the like. In addition, in the hollow needle 1 of this example, the proximal end of the respective lateral holes 7a, 7b, and 7c and the proximal end of the distal end section 3 are spaced away from each other, and the respective lateral holes 7a, 7b, and 7c do not extend to the proximal end of the distal end section 3.

In addition, the medical resin-made hollow needle 1 of this example includes grooves 11a, 11b, and 11c which respectively communicate with the lateral holes 7a, 7b, and 7c, and extend to the puncture end portion 2 of the distal end section 3. In this example, the grooves 11a, 11b, and 11c extend in parallel to the central axis of the hollow needle 1 in an approximately the same width, and the distal ends of the grooves 11a, 11b, and 11c are located at the center of the puncture end portion 2. As illustrated in Fig. 6, the lateral holes 7 (7a, 7b, and 7c) communicate with the lumen-like portion 15 and the outside. In this example, as illustrated in Fig. 6, the lateral holes 7 (7a, 7b, and 7c) are formed to communicate with a distal end of the lumen-like portion 15, and are opened toward a direction that is perpendicular to the central axis of the hollow needle 1.

The large-diameter section 4 is a cylindrical section that extends from a distal end of the hub section 5 in a distal end direction. The large-diameter section 4 has the length N of 4 to 12 mm, and the distal end outer diameter Q greater than the maximum outer diameter R of the distal end section 3 by 0.6 to 3.0 mm. When having the length and the outer diameter, it is possible to maintain a stable connection state into the port 63 of the infusion solution container 70. Furthermore, it is preferable that the length N of the large-diameter section 4 is preferably 5 to 10 mm, and more preferably 6 to 9 mm. In addition, it is preferable that the distal end outer diameter Q of the large-diameter section 4 is greater than the maximum outer diameter R of the distal end section 3 by 0.8 to 2.5 mm, and more preferably 1.0 to 2.0 mm. Furthermore, in the hollow needle 1 of this example, the distal end outer diameter Q is 3.1 to 6.0 mm. In addition, the large-diameter section 4 has a gently-sloping taper angle, and a proximal end thereof has the maximum diameter. It is preferable that a taper of the large-diameter section is approximately 0.8% to 3.5% (0.5° to approximately 2°). According to this, it is possible to maintain a stable connection state into the port 63 of the infusion solution container 70, and puncturing of the elastic port-sealing member 74 of the port 63 becomes easier. Furthermore, the large-diameter section may not substantially have a taper angle .

In the tapered section 6 that connects the proximal end of the distal end section 3 and the distal end of the large-diameter section 4, a length is set to 0.3 to 4.7 mm, and a taper angle (taper expansion angle) is set to 10° to 50°. When the distal end outer diameter Q of the large-diameter section 4 is greater than the maximum outer diameter R of the distal end section 3 by 0.6 to 3.0 mm, and the tapered section has the length and the taper angle, the tapered section 6 can be easily inserted through the elastic sealing member of the port of the infusion solution container in succession to the distal end section 3, and the large-diameter section can enter the inside of the elastic sealing member. In addition, the tapered section 6 is resistant against the puncturing of the elastic sealing member 42 of the vial container 40 with the hollow needle 1, and thus it is possible to easily stop the puncturing by the hollow needle 1. Furthermore, it is preferable that the length of the tapered section 6 is 1.3 to 2.5 mm. In addition, it is preferable that the taper angle (taper expansion angle) is 15° to 30°, and more preferably 20° to 25°.

The hub section 5 is a cylindrical section that backwardly extends from the proximal end of the large-diameter section 4, and has an inner surface shape that can be attached to a Luer-taper nozzle of a medical device. Specifically, the inside of the hub section 5 has a 6% taper, and a medical female-type connection portion (for example, a nozzle portion of a syringe) with a typical standard having a 6% taper can be connected to the inside. The medical female-type connection portion of a typical standard represents "conical fittings with a 6% (approximately 3.43°) taper for syringes, needles, and certain other medical device-Part 1: general requirements" in ISO594-1:1986.

In addition, in the medical resin-made hollow needle 1, two ribs 8a and 8b, which are provided to face each other, are provided on an outer surface of the proximal end portion of the hub section 5. The ribs 8a and 8b can be used as a portion to be screwed when being screwed with a threaded portion of another medical device. In addition, in the hollow needle 1 of this example, protrusions 9a and 9b, which extend in an axial direction, are provided on the outer surface of the hub section 5. The protrusions can be used as a grip portion of the hollow needle 1, and it is easy to screw the hub section 5 with another medical device due to the protrusions.

In addition, the medical resin-made hollow needle 1 includes a hub-section distal end portion 5a that is provided at a distal end of the hub section 5 and has a slightly small diameter, and a stepped portion 5b that is provided at a distal end of the hub-section distal end portion 5a. The stepped portion 5b functions as a locking portion that stops puncturing of the medical resin-made hollow needle 1 upon connection to the port of the infusion solution container. In this example, the stepped portion 5b is configured as a sharply-diameter-expanding tapered portion in which a total length is short and a diameter sharply expands toward a rear side.

As a formation material of the medical resin-made hollow needle 1, for example, thermoplastic resins such as polypropylene, polyethylene, polystyrene, polyamide, polycarbonate, polyvinyl chloride, poly-(4-methylpentene-1), an acrylic resin, an acrylonitrile-butadiene-styrene copolymer, polyesters such as polyethylene terephthalate, a cyclic olefin-based resin, polyether ether ketone, polybutylene terephthalate, polycarbonate, polyamide, polyacetal, modified polyphenylene ether, a polyester-based resin, a fluorine-based resin, polysulfone, polyether imide, polyether sulfone, polyether ketone, polyether lactone, and liquid crystal polyester, or thermosetting resins such as an epoxy resin, an unsaturated polyester resin, a phenol resin, a urea resin, a melamine resin, and a polyurethane resin, and the like can be used.

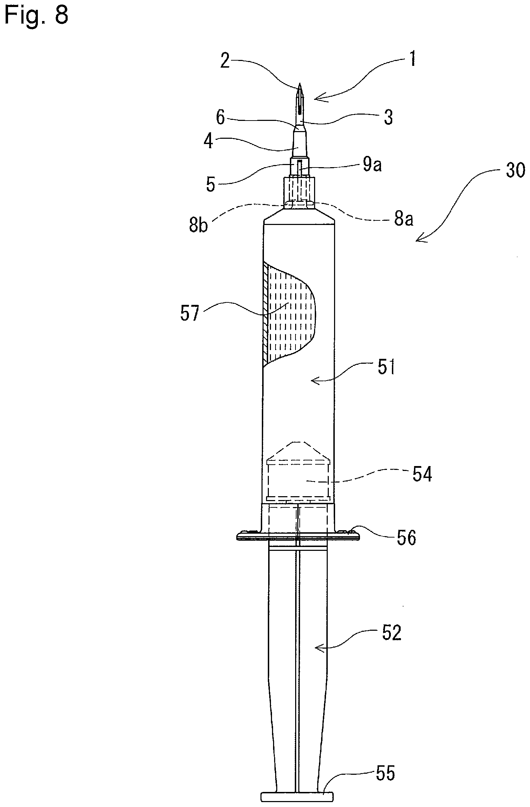

Next, description will be given of an operation of the medical resin-made hollow needle 1 of the invention with reference to Fig. 8 to Fig. 11.

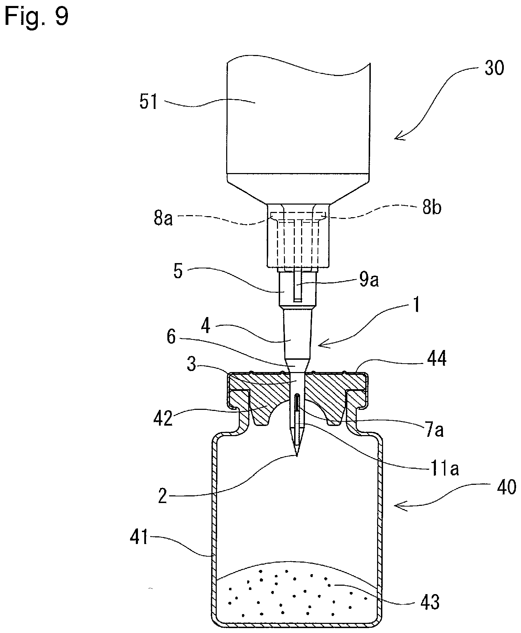

When using the medical resin-made hollow needle 1 of the invention, a syringe 30 filled with a medical liquid 57 such as water for injection which dissolves or dilutes a drug as illustrated in Fig. 8, a vial container in which a drug 43 is accommodated as illustrated in Fig 9, and the infusion solution container 70 filled with an infusion solution 71 are prepared.

In addition, as illustrated in Fig. 8, the medical resin-made hollow needle 1 is attached to a nozzle portion of the syringe 30 filled with the medical liquid 57 by using the hub section 5. Furthermore, the syringe 30 that is used here includes an outer cylinder 51, a gasket 54 that is slidable at the inside of the outer cylinder in a liquid-tight body, and a plunger 52 that is connected to the gasket 54. In addition, the outer cylinder 51 includes a nozzle portion provided at a distal end and a flange portion 56 provided at a lower end. In addition, the plunger 52 includes a gasket mounting portion provided at a distal end, and a pressing portion 55 provided at a proximal end.

In addition, as illustrated in Fig. 9, the syringe 30, to which the medical resin-made hollow needle 1 of the invention is connected, punctures the elastic sealing member 42 of the vial container 40 by using the distal end section 3, which includes the puncture end portion 2, of the hollow needle 1. Due to the puncturing, the puncture end portion 2 penetrates through an encapsulation member 44 and the elastic sealing member 42, and the distal end of the puncture end portion 2 reaches the inside of a main body 41 of the vial container 40. In addition, as illustrated in Fig. 9, when the distal end of the tapered section 6 reaches the ceiling surface of the elastic sealing member 42 of the vial container, the puncturing operation into the vial container 40 is completed. At this time, the tapered section 6 is resistant against the puncturing, and thus a user can easily grasp completion of the puncturing operation into the vial container 40.

In addition, the distal end section 3, which includes the puncture end portion 2, of the hollow needle 1 has a sufficient length, and thus in completion of the puncturing into the vial container, the puncture end portion can be confirmed from the outside. In addition, at this time, the lateral holes 7 (7a, 7b, and 7c) communicate with the inside of the vial container 40. In addition, the plunger 52 is pressed to inject the medical liquid 57 inside the syringe 30 into the vial container 40. At this time, the lateral holes 7 (7a, 7b, and 7c) are opened in a direction perpendicular to the central axis of the hollow needle 1, and thus the momentum of the medical liquid 57, which is injected into the vial container 40, is dispersed in a transverse direction. According to this, it is possible to prevent the medical liquid 57 from vigorously colliding with the drug and foaming from occurring.

In addition, after dissolving or diluting the drug 43 in the vial container 40 with the medical liquid 57, the vial container 40 is vertically inverted together with the syringe 30, and the plunger 52 is pulled to suction a medical solution, which is obtained through dissolving or dilution of the drug 43, into the syringe 30. At this time, the lateral holes 7 (7a, 7b, and 7c) are located in the vicinity of an inner surface of the elastic sealing member 42 of the vial container 40, and thus it is possible to omit an operation of matching the position of the lateral holes 7 (7a, 7b, and 7c) with the inner surface of the elastic sealing member 42 so as to suction the medical solution. After suctioning the medical solution into the syringe 30, the syringe 30, to which the hollow needle 1 is connected, is detached from the vial container 40.

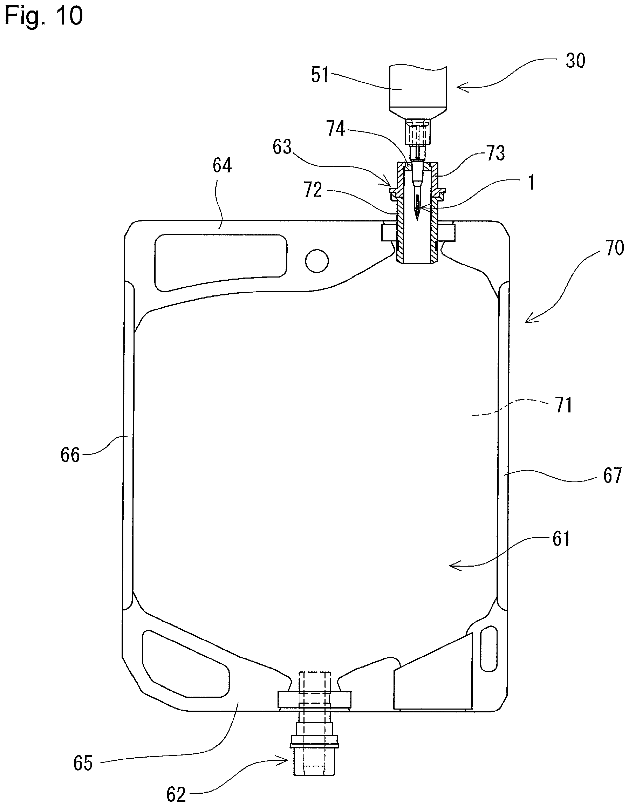

Continuously, even in the case of administering the medical solution in a state of being mixed with the infusion solution 71, as illustrated in Fig. 10, the syringe 30 filled with the medical solution is connected to the mixed injection port 63 of the infusion solution container 70. Furthermore, the infusion solution container 70 that is used here includes a pouch-like container main body 61 including an upper sealing portion 64, a lower sealing portion 65, and lateral sealing portions 66 and 67, the mixed injection port 63 that is fixed to the upper sealing portion 64 and communicates with the inside of the container main body, and a discharge port 62 that is fixed to the lower sealing portion 65 and communicates with the inside of the container main body. In addition, the mixed injection port 63 includes a cylindrical member 72, and a cap 73 including the elastic sealing member 74. An upper opening of the cylindrical member 72 is sealed with the cap 73.

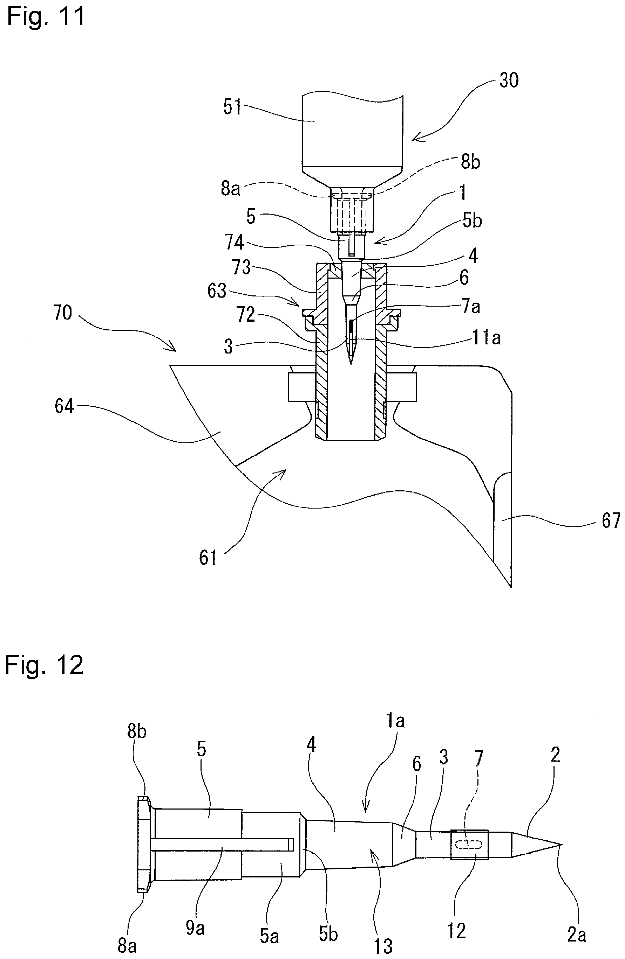

An operation of connecting the syringe 30, which is filled with the medical solution, to the mixed injection port 63 is performed by puncturing the elastic sealing member 74 of the mixed injection port 63 with the distal end section 3, which includes the puncture end portion 2, of the hollow needle 1 as illustrated in Fig. 11. Through the puncturing, the puncture end portion 2 penetrates through the elastic sealing member 74, and the distal end thereof reaches the inside of the mixed injection port 63. In addition, when the syringe 30 is pressed, the distal end section 3 of the hollow needle 1 penetrates through the elastic sealing member 74 and reaches the inside of the mixed injection port 63.

In addition, when the syringe is pressed, it enters a state in which the tapered section 6 penetrates through the elastic sealing member 74 and reaches the inside of the mixed injection port 63, and the large-diameter section 4 is located at the inside of the elastic sealing member 74. In addition, it enters a state in which the large-diameter section 4 is fixed to the inside of the elastic sealing member 74 due to an elastic force of the elastic sealing member 74, and the hollow needle 1 is held to the mixed injection port 63. In this state, it is possible to inject the medical solution into the infusion solution container by pressing the plunger 52. After injection of the medical solution is terminated, the syringe 30, to which the hollow needle 1 is attached, is detached from the infusion solution container.

Furthermore, in the case of administering the medical solution into a vein through an infusion solution line without being mixed with the infusion solution, after suctioning the medical solution into the syringe 30, the hollow needle 1 is removed from the syringe 30, and the nozzle portion of the syringe 30 is connected to the mixed injection port of the infusion solution line, and the medical solution is injected by operating the plunger 52. Furthermore, in a case where the mixed injection port can be punctured, the hollow needle 1, which is attached to the syringe 30, is connected to the mixed injection port. In addition, in the case of directly administering the medical solution to the vein, a subcutis, or the like, after suctioning the medical solution into the syringe 30, the hollow needle 1 is removed from the syringe 30, and a syringe needle is connected to the nozzle portion of the syringe 30. In this state, the syringe needle punctures the vein or the subcutis, and then the medical solution is administered by operating the plunger 52.

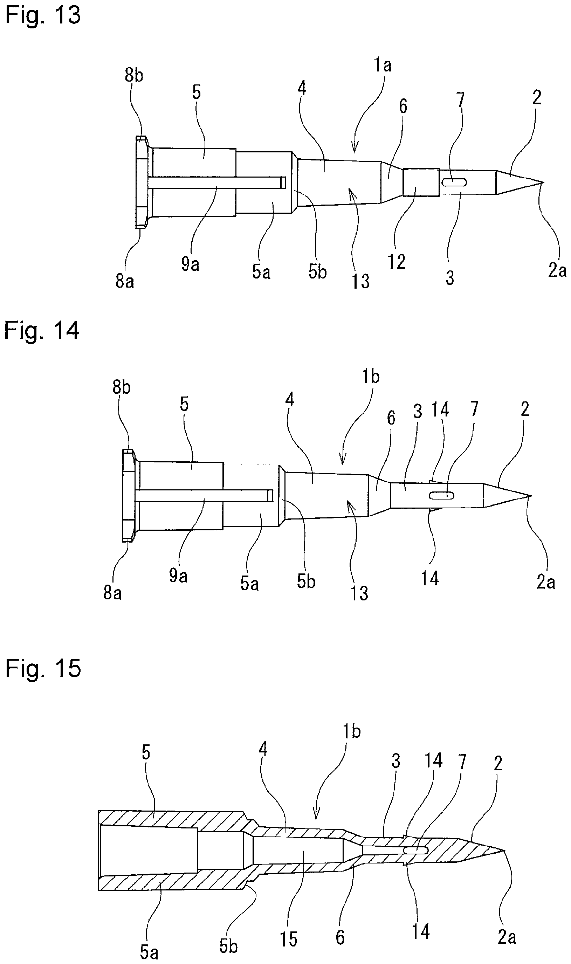

Next, description will be given of a medical resin-made hollow needle 1a of an example illustrated in Fig. 12 and Fig. 13.

A basic configuration of the medical resin-made hollow needle 1a of this example is the same as that of the medical resin-made hollow needle 1 of the above-described example. A great difference is in that a cylindrical cover member 12, which encapsulates a lateral hole 7, is provided.

The medical resin-made hollow needle 1a of this example includes a hollow needle main body 13 and the cylindrical cover member 12. As is the case with the hollow needle 1, the hollow needle main body 13 includes a distal end section 3 including a puncture end portion 2, a large-diameter section 4 that has a diameter greater than that of the distal end section 3 and a predetermined length, a short tapered section 6 that is provided between the large-diameter section 4 and the distal end section 3, a hub section 5 that is provided in a proximal end and can be attached to a Luer-taper nozzle of a medical device, and the cylindrical cover member 12 that encapsulates the lateral hole 7.

In the hollow needle 1a of this example, the hollow needle main body 13 does not include the grooves which are provided in the above-described hollow needle 1 to communicate with the lateral holes 7. Accordingly, a distal end outer edge of the lateral hole 7 is exposed on an outer surface of the hollow needle main body 13. In addition, the cylindrical cover member 12 is configured to encapsulate and substantially seal an outer surface of the lateral hole 7. In addition, as illustrated in Fig. 13, the cylindrical cover member 12 is configured to be movable (specifically, to be slidable) at least toward a proximal end side (in order words, in a direction toward the tapered section 6). In addition, exposure of the lateral hole 7 is permitted due to the movement toward the proximal end side.

The cylindrical cover member 12 has at least a length in an axial direction that is longer than a length of the lateral hole in an axial direction, and an inner diameter of the cylindrical cover member 12 is set to be approximately the same as the maximum outer diameter of the distal end section 3. In addition, it is preferable that a movable distance of the cylindrical cover member 12 is approximately the same as a total length of the cylindrical cover member . As the cylindrical cover member, a thin rigid synthetic resin tube can be appropriately used. As a formation material of the cylindrical cover member, polyethylene, polypropylene, nylon, polyethylene terephthalate, polyimide, fluorine-based polymers such as PTFE and ETFE, an olefin-based polymer, silicone, and the like are preferred.

Next, description will be given of a medical resin-made hollow needle 1b of an example illustrated in Fig. 14 and Fig. 15.

A basic configuration of the medical resin-made hollow needle 1b of this example is the same as that of the medical resin-made hollow needle 1 of the above-described example. A great difference is in that a protrusion 14 (a locking protrusion or a separation regulating protrusion) that protrudes from an outer surface in the vicinity of a lateral hole 7. The protrusion 14 is a locking protrusion. In order words, the protrusion 14 functions as a separation regulating protrusion.

In addition, in the medical resin-made hollow needle 1b of the example illustrated in Fig. 14 and Fig. 15, the protrusion 14 protrudes from an outer surface of the distal end section 3 at a position on a lateral side of the lateral hole 7. Specifically, the protrusion 14 is provided at a position that is slightly spaced away from a lateral portion of the lateral hole 7 in a peripheral direction. Particularly, in the hollow needle 1b of this example, two lateral holes 7 are provided to face each other, and two protrusions 14 are provided to face each other at central positions between the two lateral holes 7. Accordingly, the two lateral holes 7 and the two protrusions 14 are arranged to be spaced away from each at an equal angle with respect to the central axis of the distal end section 3. Furthermore, the protrusions 14 may be formed to be close to the lateral holes 7, that is, at a lateral portion of the lateral holes 7.

Furthermore, the number of the lateral holes 7 is not limited to two pieces as described above, and three pieces may be provided similar to the above-described example. In addition, the number of the protrusions 14 is not limited to two pieces as described above, and one piece or three pieces maybe provided. Particularly, in the case where three lateral holes are provided, the protrusions 14 may be respectively provided between the lateral holes, and in this case, three protrusions are provided. In addition, in the case of providing three or greater protrusions 14, it is preferable that the protrusions 14 are arranged at an equal angle with respect to the central axis of the distal end section 3.

In addition, it is preferable that the protrusions 14 include an inclined portion of which a height gradually increases from a distal end toward a proximal end as illustrated in Fig. 14 and Fig. 15. In addition, it is preferable that the proximal end portion of the protrusions 14 includes an erecting portion. Particularly, it is preferable that the erecting portion is an erecting surface that is perpendicular to the central axis of the distal end section 3. In addition, a width of the protrusions 14 may gradually increase from the distal end toward the proximal end. In addition, in the protrusions 14, a width of a portion on the distal end side gradually increases toward the proximal end, and a portion on the proximal end side may extend to the proximal end with approximately the same width.

In addition, it is preferable that the proximal end of the protrusions 14 may approximately match the proximal end of the lateral holes 7 or may be located on a slightly distal end side with respect to an axial direction of the distal end section 3.

When the protrusions are provided, during puncturing into the elastic container-sealing member of the vial container, if the protrusions pass through the elastic sealing member, insertion resistance slightly decreases, and thus an operator can feel arrival of the lateral holes 7 into the vial container. In addition, the protrusions 14 can prevent unintended detachment of the hollow needle 1b from the vial container after puncturing. In addition, after puncturing the elastic container-sealing member with the hollow needle 1b, when the proximal end of the protrusions 14 are brought into contact with an inner surface of the elastic container-sealing member, the lateral holes 7 are located in the vicinity of the inner surface of the elastic container-sealing member. Accordingly, after dissolving or diluting the drug inside the vial container with the medical liquid, it is possible to simply match the position of the lateral holes 7 with the inner surface of the elastic container-sealing member.

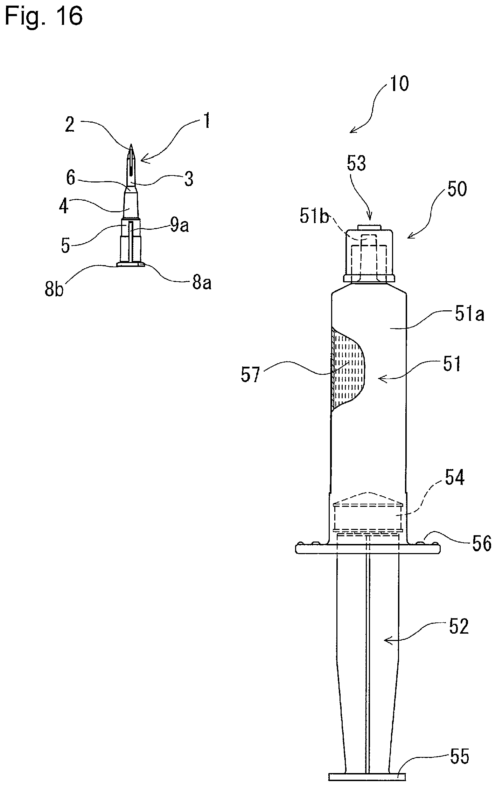

Next, description will be given of a medical device set 10 of the invention according to an example illustrated in Fig. 16.

The medical device set 10 of this example includes the medical resin-made hollow needle 1 and a medical device 50.

As a medical device, a pre-filled syringe 50 is used. The pre-filled syringe 50 includes an outer cylinder 51 including an outer cylinder main body 51a having a cylindrical shape and a Luer-taper nozzle 51b that is provided at a distal end portion of the outer cylinder main body 51a and has a distal end opening, a gasket 54 that is slidably accommodated in the outer cylinder 51, a medical liquid 57 that is filled the in the outer cylinder 51, a sealing member 53 that seals the distal end opening, and a plunger 52 that is attached to a proximal end of the gasket 54 or is attachable thereto.

In the medical device set 10 that is illustrated in the drawing, the medical resin-made hollow needle 1 is used, but as the medical resin-made hollow needle, the medical resin-made hollow needles in the entirety of the above-described examples can be used.

As the pre-filled syringe 50, a known syringe is used.

Examples of the medical liquid 57 that is filled in the pre-filled syringe 50 include a dissolving solution or a diluting solution such as water for injection, physiological saline, and aqueous glucose solution, and a medical solution that contains a drug (for example, vitamins, minerals, cyclosporine, a benzodiazepine-based drug, a sodium-chloride injection solution, antibiotics, and a protein preparation) and can dissolve or dilute a powdery preparation inside the vial container which is a use target.

The pre-filled syringe 50 includes the sealing member 53 (specifically, a detachable cap) that is attached to the Luer-taper nozzle 51b so as to seal a distal end opening of the outer cylinder 51. As the sealing member, apeelable film-shaped member, which seals a distal end surface of an inner cylinder portion 23 of a nozzle portion 22, is also possible.

The outer cylinder 51 is a cylindrical body that is formed from a transparent or translucent material, as necessary, a material that has small oxygen permeability and water vapor permeability. The outer cylinder 51 includes the main body 51a, the Luer-taper nozzle 51b, and a flange 56 that is provided at a rear end portion. The Luer-taper nozzle 51b is configured as a medical female-type connection portion with a typical standard. The medical female-type connection portion of a typical standard represents "conical fittings with a 6% (approximately 3.43°) taper for syringes, needles, and certain other medical device-Part 1: general requirements" in ISO594-1:1986.

As illustrated in Fig. 16, the gasket 54 includes a main body that extends with approximately the same outer diameter, and a plurality of annular ribs (two pieces in this example) which are provided in the main body, and the gasket 54 slides while coming into contact with an inner surface of the outer cylinder 51 in an air-tight manner.

In addition, the plunger 52 is connected to a rear end portion of the gasket 54. The plunger 52 may be attached in use. The plunger 52 includes a pressing portion 55 at a rear end thereof.

In addition, the medical device set of the invention may include a vial container which accommodates a drug that is dissolved or diluted with the medical liquid 57 that is filled in the pre-filled syringe 50. As the vial container, the above-described vial container 40 illustrated in Fig. 9 is appropriately used. In addition, the medical device set of the invention may include the vial container that accommodates the drug that is dissolved or diluted with the medical liquid 57 in the pre-filled syringe 50, and an infusion solution container that accommodates an infusion solution with which a medical solution obtained by dissolving or diluting a drug is mixed. As the infusion solution container, the above-described infusion solution container 70 illustrated in Fig. 10 can be appropriately used.

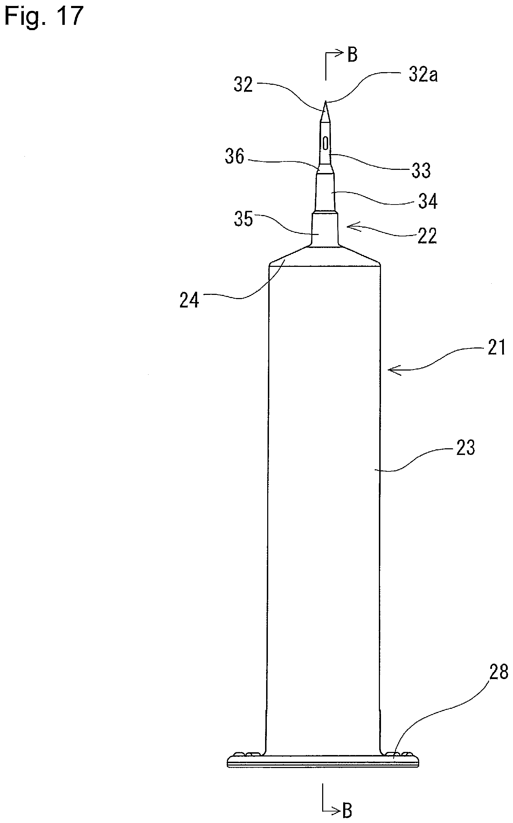

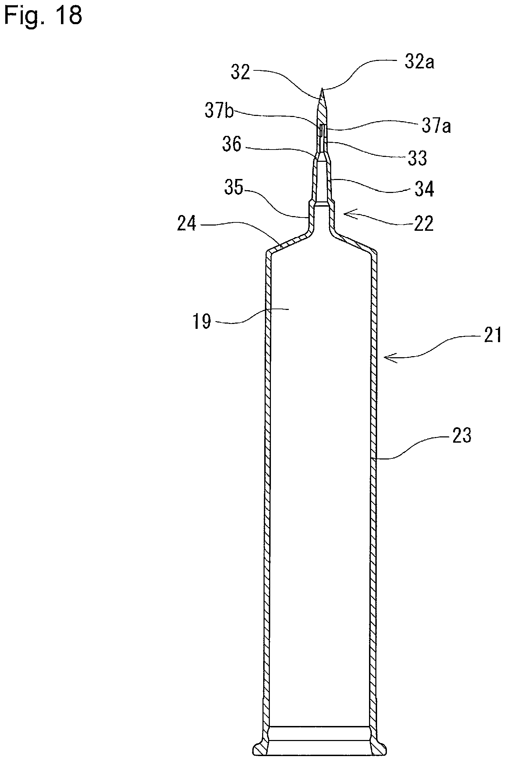

Next, description will be given of an outer cylinder with puncture portion according to the invention with reference to Fig. 17 to Fig. 19.

As illustrated in Fig. 17 to Fig. 19, the outer cylinder with puncture portion 21 of the invention includes an outer cylinder main body 23 having a cylindrical shape, and a hollow puncture portion 22 that is provided at a distal end portion of the outer cylinder main body 23. The puncture portion 22 includes a distal end section 33 including a puncture end portion 32, a large-diameter section 34 having a diameter greater than that of the distal end section 33 and a predetermined length, and a short tapered section 36 that is provided between the large-diameter section 34 and the distal end section 33. The distal end section 33 includes holes 37a and 37b which are provided on a proximal end side in comparison to the distal end 32a by a predetermined length and a distal end side in comparison to the tapered section 36 by a predetermined length, and communicates with the inside and the outside of the puncture portion 22, and a tapered puncture end portion 32.

In addition, a total length of the distal end section 33 is 7 to 16 mm, and a maximum outer diameter thereof is 1.8 to 2.4 mm. A distance between a distal end of the lateral holes 37a and 37b and a rear end of the distal end section 33 is 3.5 mm or greater. A length of the lateral holes 37a and 37b in an axial direction is 1.0 to 2.5 mm. In the tapered section 36, a length of the large-diameter section 34 is 6 mm or greater and an average outer diameter is 3.0 to 4.0 mm. In addition, the distal end section 33 can be inserted through the elastic sealing member 42 of the vial container 40. The distal end section 33 and the tapered section 36 can be inserted through the elastic sealing member 74 of the port 63 of the infusion solution container 70. The large-diameter section 36 can penetrate into the elastic sealing member 74 of the port 63 of the infusion solution container 70, and a state, in which the large-diameter section 34 has entered the inside of the elastic sealing member 74, can be maintained.

A basic configuration of the hollow puncture portion 22 in the outer cylinder with puncture portion 21 of this example is the same as that of the above-described medical resin-made hollow needle 1.

Description will now be given of the hollow puncture portion 22 in the outer cylinder with puncture portion 21 of this example.

The hollow puncture portion 22 includes the distal end section 33 including the puncture end portion 32, the large-diameter section 34 having a diameter greater than that of the distal end section 33 and a predetermined length, the short tapered section 36 that is provided between the large-diameter section 34 and the distal end section 33, and a base section 35 that connects a shoulder portion 24 of the outer cylinder main body 23 and the large-diameter section 34. In addition, a lumen-like portion of the hollow puncture portion 22 communicates with an internal space 19 of the outer cylinder main body 21.

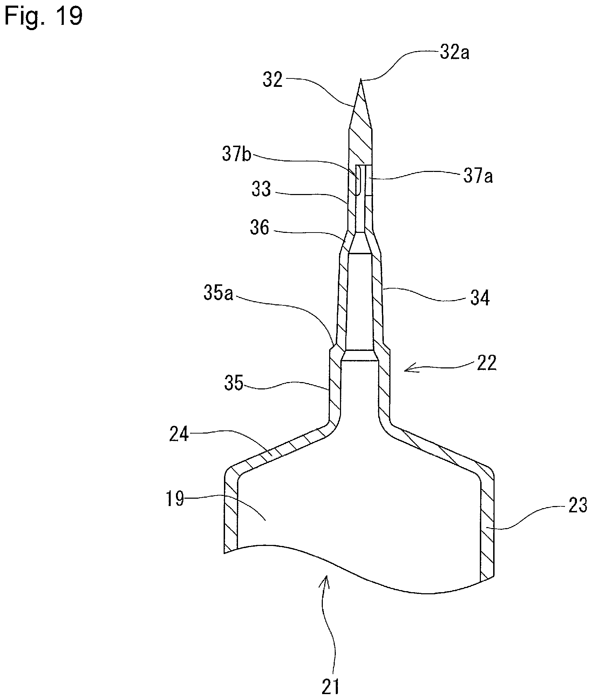

A shape of the distal end section 33 is the same as that of the distal end section 3 of the medical resin-made hollow needle 1. Furthermore, as illustrated in Fig. 19, the lateral holes 37a and 37b communicate with the internal space 19 of the puncture outer cylinder 21 through the lumen-like portion of the hollow puncture portion 22 and the outside.

In addition, even in the outer cylinder with puncture portion 21 of this example, as is the case with the above-described medial resin-made hollow needle 1a, a cylindrical cover member that encapsulates the lateral holes maybe provided. In addition, as the cylindrical cover member, a cover member, which can move (specifically, slide) at least toward the tapered section 36, and can permit exposure of the lateral holes through the movement, is used.

The large-diameter section 34 is a cylindrical section that extends from the distal end of the base section 35 toward the distal end. A shape of the large-diameter section 34 is the same as that of the large-diameter section 4 of the above-described medical resin-made hollow needle 1.

In addition, a shape of the tapered section 36, which connects a proximal end of the distal end section 33 and a distal end of the large-diameter section 34, is the same as that of the tapered section 6 of the above-described medical resin-made hollow needle 1.

The outer cylinder main body 23 includes a cylindrical main body, the shoulder portion 24 that is located at a distal end portion, and a flange portion 28 that is provided at the proximal end. In addition, as described above, the outer cylinder main body 23 and the hollow puncture portion 22 are connected by the base section 35. The base section 35 is configured as a cylindrical section having a diameter greater than that of the large-diameter section 34, and a stepped portion 35a is formed between the base section 35 and the large-diameter section 34. The stepped portion 35a is formed in a tapered shape in which a length is short and a diameter sharply expands.

Examples of a formation material of the outer cylinder with puncture portion 21 of the invention include various kinds of resins such as polypropylene, polyethylene, polystyrene, polyamide, polycarbonate, polyvinyl chloride, poly-(4-methylpentene-1), an acrylic resin, an acrylonitrile-butadiene-styrene copolymer, polyester including polyethylene terephthalate, a cyclic polyolefin polymer, and a cyclic olefin copolymer. Among these, resins such as the polypropylene, the cyclic polyolefin polymer, and the cyclic olefin copolymer are preferable because of their ease of molding and heat resistance. Furthermore, as a formation material of the outer cylinder with puncture portion 21, the cyclic olefin polymer and the cyclic olefin copolymer, which have high transparency to a certain extent at which the medical solution on an inner side can be confirmed from an outer side with the naked eyes and heat resistance capable of enduring high-pressure steam sterilization, are particularly preferable.

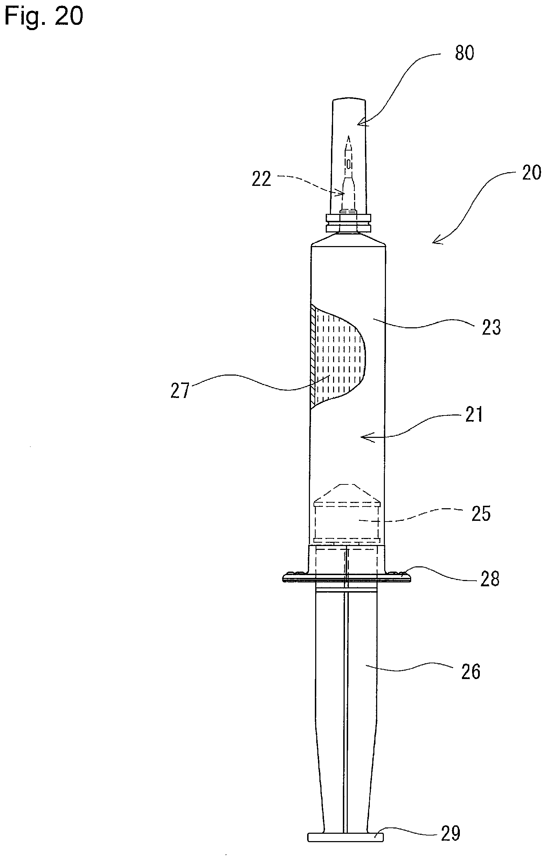

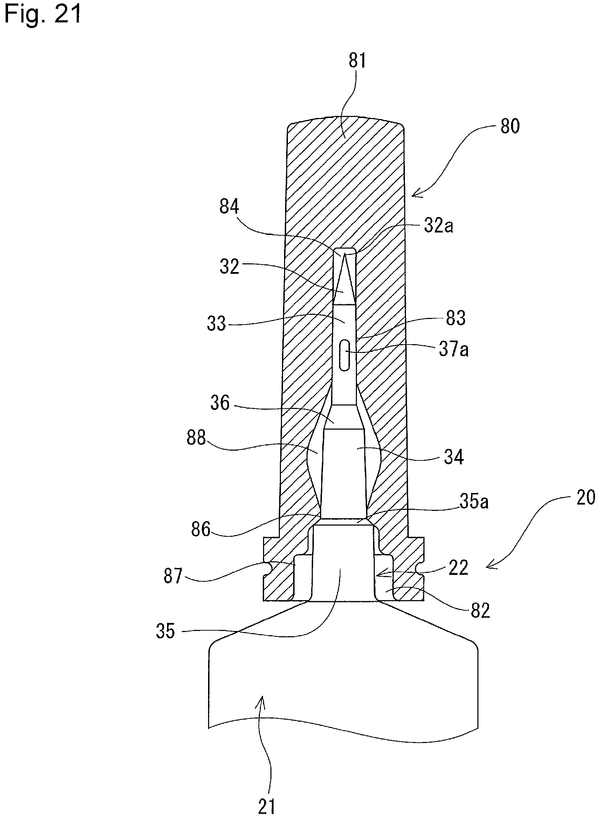

Next, description will be given of a pre-filled syringe of the invention with reference to Fig. 20 and Fig. 21.

A pre-filled syringe 20 of the invention includes an outer cylinder with puncture portion 21, a gasket 25 that is slidably accommodated in the outer cylinder with puncture portion 21, a drug 27 that is filled the in the outer cylinder with puncture portion 21, and a plunger 26 that is attached to a proximal end of the gasket 25 or is attachable thereto.

Furthermore, the pre-filled syringe 20 of this example uses the above-described outer cylinder with puncture portion 21, and includes a sealing cap 80 that liquid-tightly seals the outer cylinder with puncture portion 21.

The configuration of the outer cylinder with puncture portion 21 is as described above.

As illustrated in Fig. 20, the gasket 25 includes a main body that extends in approximately the same outer diameter, and a plurality of annular ribs (two pieces in this example, the number of the annular ribs may be appropriately set as long as liquid-tightness and sliding properties are satisfied in two pieces or greater) which are provided in the main body, and the ribs come into contact with an inner surface of the outer cylinder 3 in a liquid-tight manner. In addition, a distal end surface of the gasket 25 has a shape corresponding to a distal end inner surface of the main body 23 of the outer cylinder with puncture portion 21 so that a gap between the gasket 25 and the outer cylinder with puncture portion 21 becomes as small as possible when the distal end surface of the gasket 25 comes into contact with the distal end inner surface of the outer cylinder with puncture portion 21.

As a formation material of the gasket 25, an elastic rubber (for example, a butyl rubber, a latex rubber, and a silicone rubber), a synthetic resin (for example, a styrene-based elastomer such as an SBS elastomer and an SEBS elastomer, a polyolefin-based elastomer such as an ethylene-α olefin copolymer, and the like), and the like can be preferably used.

In addition, a concave portion, which extends from a proximal end portion toward an inner side, is provided in the gasket 25, and a plunger attaching portion is provided in the concave portion. In this example, the plunger attaching portion is constituted, for example, by a female screw portion.

The plunger 26 includes a main body that has a cross-shaped cross-section and extends in an axial direction, a gasket attaching portion that protrudes from the main body, a pressing disk portion 29 that is provided in the proximal end portion. For example, the gasket attaching portion is constituted by a protrusion and a male screw portion formed on an outer surface thereof. Furthermore, the plunger 26 may be not attached and may be attached in use.

Examples of the medical liquid 27 that is filled in the pre-filled syringe 20 include a dissolving solution or a diluting solution such as water for injection, physiological saline, and aqueous glucose solution, and a medical solution that contains a drug (for example, vitamins, minerals, cyclosporine, a benzodiazepine-based drug, a sodium-chloride injection solution, antibiotics, and a protein preparation) and can dissolve or dilute a powdery preparation inside the drug container which is a use target.

Next, description will be given of the sealing cap 80 with reference to Fig. 21.

The sealing cap 80 of this example includes a closed distal end portion 81, an opened proximal end portion 82, and a puncture portion accommodating portion 88 that accommodates a hollow puncture portion 22 of the outer cylinder with puncture portion 21. In addition, the puncture portion accommodating portion 88 includes a distal end accommodating portion 84 that accommodates the puncture end portion 32, a sealing portion 83 that accommodates the distal end section 33 of the puncture portion 22 and seals the lateral hole 37a provided in the small-diameter section 33, a large-diameter section accommodating portion that accommodates the large-diameter section 34, a locking portion 86 that engages with the stepped portion 35a, and a proximal end portion 87 having an expanded diameter.

In addition, when the sealing cap 80 is mounted to the hollow puncture portion 22 of the outer cylinder with puncture portion 21, the lateral hole 37a of the hollow puncture portion 22 is sealed by the sealing portion 83 and comes into close contact with the sealing portion 83, and the stepped portion 35a and the locking portion 86 engage with each other and enter a close contact state.

Specifically, the sealing cap 80 includes the locking portion 86 that is provided on an inner surface located at a position on a distal end side in comparison to the opened proximal end portion 82 by a predetermined length and protrudes in an annular shape. The locking portion 86 includes a vertex portion that mostly protrudes, a rear side inclined portion (tapered portion) of which a protrusion height gradually decreases from the vertex portion toward an opened proximal end portion direction, and a front side inclined portion (tapered portion) of which a protrusion height gradually decreases from the vertex portion toward a distal end direction.

In addition, in the sealing cap 80 of this embodiment, the sealing portion 83, which accommodates the distal end section 33 and seals the lateral hole 37a provided in the small-diameter section 33, is configured to have an inner diameter that is approximately the same as or slightly smaller than an outer diameter of the distal end section 33, and a cylindrical inner surface that extends in an axial direction by a predetermined length. A protrusion, which has a size capable of being inserted into the lateral hole 37a, may be provided in the sealing portion 83. The protrusion has an effect of preventing a solution from being leaked from the lateral hole 37a.

In addition, in this example, in a state in which the stepped portion 35a of the outer cylinder with puncture portion 21 and the locking portion 86 of the sealing cap 80 engage with each other, the hollow puncture portion 22 of the outer cylinder with puncture portion 21 does not proceed from the state . In addition, in a state in which the stepped portion 35a and the locking portion 86 engage with each other, the distal end 32a of the puncture end portion 32 of the hollow puncture portion 22 does not reach a distal end surface of the distal end accommodating portion 84.

As a formation material of the sealing cap 80, it is necessary for at least the puncture portion accommodating portion 88 to be formed from an elastic material into which a puncture needle can be inserted. As the elastic material into which the puncture needle can be inserted, for example, rubbers such as a butyl rubber, an isoprene rubber, a latex rubber, and a silicone rubber, elastomers such as a synthetic resin elastomer (for example, a styrene-based elastomer such as an SBS elastomer and an SEBS elastomer, and an olefin-based elastomer such as an ethylene-α olefin copolymer elastomer, and the like), and the like are preferable.

In the medical resin-made hollow needle of the above-described examples, the holes which communicate with the inside and the outside are not limited to the above-described lateral holes, and may be a distal end opening, a combination of a lateral hole and a distal end opening, a plurality of minute lateral holes, and the like are also possible.

### Industrial Applicability

An aspect of the medical resin-made hollow needle of the invention is as follows.
(1) A medical resin-made hollow needle, comprising:
   a distal end section including a puncture end portion at a distal end thereof;
   a large-diameter section having a diameter greater than a diameter of the distal end section, and a predetermined length;
   a tapered section that is provided between the large-diameter section and the distal end section, and has a diameter that increases toward a proximal end thereof; and
   a hub section that is provided in a proximal end of the hollow needle, and is attachable to a Luer-taper nozzle of a medical device,
   wherein the distal end section includes a hole that communicates with an inside and an outside of the resin-made hollow needle,
   wherein the distal end section has a total length in a range of 7 to 14 mm, and a maximum outer diameter in a range of 1.5 to 3.0 mm,
   wherein the tapered section has a length in a range of 0.3 to 4.7 mm, and a taper angle in a range of 10° to 50°,
   wherein the large-diameter section has a length in a range of 4 to 12 mm, and a distal end outer diameter that is greater than the maximum outer diameter of the distal end section by a range of 0.6 to 3.0 mm,
   wherein the distal end section is configured to pierce an elastic container-sealing member that seals an opening of a vial container, and the distal end section and the tapered section are configured to pierce an elastic port-sealing member that seals an opening of a port of an infusion solution container, and
   wherein the large-diameter section is configured to enter the elastic port-sealing member, and a state of the large-diameter section being entering into the elastic port-sealing member is configured to be maintained due to a pressure applied from the elastic port-sealing member.

   In the medical resin-made hollow needle, the distal end section is sufficiently thin while maintaining strength, and has a length to a certain extent. Accordingly, connection to the vial container through puncturing is easy, and it is also possible to confirm a puncture end portion in the puncturing. Accordingly, it is possible to perform injection of a liquid into the vial container in an easy and reliable manner. In addition, in the medical resin-made hollow needle, the tapered section and the large-diameter section are configured as described above, and thus in connection to the elastic sealing member of the port of the infusion solution container, insertion of the tapered section through the elastic sealing member, and insertion of the large-diameter section into the elastic sealing member are possible, and the inserted large-diameter section is reliably maintained by the elastic sealing member of the infusion solution container. According to this, in the medical resin-made hollow needle, a connection state to the port of the infusion solution container is maintained in a stable state.
   In addition, the aspect may be as follows.
(2) The medical resin-made hollow needle according to (1),
   wherein the puncture end portion includes a solid tapered puncture end that has an outer diameter decreasing toward a distal end thereof, and the hole is a lateral hole that is provided proximal of the distal end of the distal end section, and is provided distal of a proximal end of the distal end section by a distance of a range of 1.8 to 5 mm.
(3) The medical resin-made hollow needle according to (2),
   wherein the distal end section includes a protrusion that protrudes from an outer surface lateral of the lateral hole, and a proximal end of the protrusion is disposed a position approximately corresponding to or slightly distal of a proximal end of the lateral hole with respect to an axial direction of the distal end section.
(4) The medical resin-made hollow needle according to (2) or (3),
   wherein a distance between a distal end of the lateral hole and the proximal end of the distal end section is in a range of 1.8 to 5.0 mm.
(5) The medical resin-made hollow needle according to any one of (1) to (4),
   wherein the puncture end portion has a conical shape having a total length in a range of 2.1 to 4.0 mm, and a taper angle in a range of 14° to 25°.
(6) The medical resin-made hollow needle according to any one of (1) to (5),
   wherein the large-diameter section has a tapered shape that has an outer diameter increasing toward a proximal end thereof in a ratio in a range of 0.8% to 3.5%.
(7) The medical resin-made hollow needle according to any one of (1) to (6), further comprising:
   a rib that is provided on an outer surface of a proximal end portion of the hub section and is configured to be screwable with a threaded portion of the medical device; and
   a protrusion that is provided on an outer surface of the hub section and extends in an axial direction.

   In addition, as aspect of a medical device set of the invention is as follows.
(8) A medical device set, comprising:
   the medical resin-made hollow needle according to any one of (1) to (7); and
   a medical device,
   wherein the medical device is a pre-filled syringe that comprises an outer cylinder that includes an outer cylinder main body having a cylindrical shape and the Luer-taper nozzle provided at a distal end portion of the outer cylinder main body and including a distal end opening, a gasket that is slidably accommodated in the outer cylinder, a medical liquid that is filled in the outer cylinder, a sealing member that seals the distal end opening of the outer cylinder, and a plunger that is attached or attachable to a proximal end of the gasket.

   In the medical device set, after detaching a sealing member of the pre-filled syringe, the medical resin-made hollow needle is connected to the Luer-taper nozzle of the syringe. Accordingly, it is possible to easily prepare the medical device set that can be connected to any of the vial container and the infusion solution container. In addition, the medical resin-made hollow needle has the above-described configuration. Accordingly, it is possible to perform injection of a liquid into the vial container in an easy and reliable manner, and it is possible to connect to the medical resin-made hollow needle to the port of the infusion solution container in a stable state.
   In addition, the aspect may be as follows.
(9) The medical device set according to (8), further comprising:
   the vial container that accommodates a drug configured to be dissolved or diluted with the medical liquid.

   In addition, an aspect of an outer cylinder with puncture portion of the invention is as follows.
(10) An outer cylinder with puncture portion, comprising:
   an outer cylinder main body having a cylindrical shape; and
   a hollow puncture portion that is provided in a distal end portion of the outer cylinder main body,
   wherein the puncture portion comprises a distal end section that includes a puncture end portion at a distal end thereof, a large-diameter section having a diameter greater than a diameter of the distal end section and having a predetermined length, and a short tapered section that is provided between the large-diameter section and the distal end section,
   wherein the distal end section includes a hole that communicates with an inside and an outside of the puncture portion, and the puncture end portion having a tapered shape,
   wherein the distal end section has a total length in a range of 7 to 14 mm, and a maximum outer diameter in a range of 1.5 to 3.0 mm,
   wherein the tapered section has a length of in a range 0.3 to 4.7 mm, and a taper angle in a range of 10° to 50°,
   wherein the large-diameter section has a length in a range 4 to 12 mm, and a distal end outer diameter that is greater than the maximum outer diameter of the distal end section by a range of 0.6 to 3.0 mm,
   wherein the distal end section is configured to pierce an elastic container-sealing member of a vial container, and the distal end section and the tapered section are configured to pierce an elastic port-sealing member of a port of an infusion solution container, and
   wherein the large-diameter section is configured to enter the elastic port-sealing member, and a state of the large-diameter section being entering into the elastic port-sealing member is configured to be maintained due to a pressure applied from the elastic port-sealing member.

   In the outer cylinder with puncture portion, the distal end section is sufficiently thin and has a length to a certain extent. Accordingly, connection to the vial container through puncturing is easy, and it is also possible to confirm a puncture end portion in the puncturing. According to this, it is possible to perform injection of a liquid into the vial container in an easy and reliable manner. In addition, in the medical resin-made hollow needle, the tapered section and the large-diameter section are configured as described above, and thus in connection to the elastic sealing member of the port of the infusion solution container, insertion of the tapered section through the elastic sealing member, and insertion of the large-diameter section into the elastic sealing member are possible, and the inserted large-diameter section is reliably maintained by the elastic sealing member of the infusion solution container. According to this, in the outer cylinder with puncture portion, a connection state to the port of the infusion solution container is maintained in a stable state.
   In addition, the aspect may be as follows.
(11) The outer cylinder with puncture portion according to (10),
   wherein the puncture end portion includes a solid tapered shape that has an outer diameter decreasing toward a distal end thereof, and the hole is a lateral hole that is provided proximal of the distal end of the distal end section by a predetermined length and is provided distal of a proximal end of the distal end section by a distance in a range of 1.8 to 5.0 mm.
(12) The outer cylinder with puncture portion according to (11),
   wherein a distance between a distal end of the lateral hole and the proximal end of the distal end section is in a range of 1.8 to 5.0 mm.
(13) The outer cylinder with puncture portion according to any one of (10) to (12), further including:
   a sealing cap that seals the hole.

   In addition, an aspect of a pre-filled syringe of the invention is as follows.
(14) A pre-filled syringe, including:
   the outer cylinder with puncture portion according to any one of (10) to (13);
   a gasket that is slidably accommodated in the outer cylinder;
   a medical liquid that is filled in the outer cylinder; and
   a plunger that is attached or attachable to a proximal end of the gasket.

Accordingly, as is the case with the outer cylinder with puncture portion, satisfactory connection to the vial container and a stable state to the port of the infusion solution container can be realized.

## Claims

1. A medical resin-made hollow needle, comprising:
a distal end section including a puncture end portion at a distal end thereof;
a large-diameter section having a diameter greater than a diameter of the distal end section, and a predetermined length;
a tapered section that is provided between the large-diameter section and the distal end section, and has a diameter that increases toward a proximal end thereof; and
a hub section that is provided in a proximal end of the hollow needle, and is attachable to a Luer-taper nozzle of a medical device,
wherein the distal end section includes a hole that communicates with an inside and an outside of the resin-made hollow needle,
wherein the distal end section has a total length in a range of 7 to 14 mm, and a maximum outer diameter in a range of 1.5 to 3.0 mm,
wherein the tapered section has a length in a range of 0.3 to 4.7 mm, and a taper angle in a range of 10° to 50°,
wherein the large-diameter section has a length in a range of 4 to 12 mm, and a distal end outer diameter that is greater than the maximum outer diameter of the distal end section by a range of 0.6 to 3.0 mm,
wherein the distal end section is configured to pierce an elastic container-sealing member that seals an opening of a vial container, and the distal end section and the tapered section are configured to pierce an elastic port-sealing member that seals an opening of a port of an infusion solution container, and
wherein the large-diameter section is configured to enter the elastic port-sealing member, and a state of the large-diameter section being entering into the elastic port-sealing member is configured to be maintained due to a pressure applied from the elastic port-sealing member.

2. The medical resin-made hollow needle according to claim 1,
wherein the puncture end portion includes a solid tapered puncture end that has an outer diameter decreasing toward a distal end thereof, and the hole is a lateral hole that is provided proximal of the distal end of the distal end section, and is provided distal of a proximal end of the distal end section by a distance of a range of 1.8 to 5 mm.

3. The medical resin-made hollow needle according to claim 2,
wherein the distal end section includes a protrusion that protrudes from an outer surface lateral of the lateral hole, and a proximal end of the protrusion is disposed a position approximately corresponding to or slightly distal of a proximal end of the lateral hole with respect to an axial direction of the distal end section.

4. The medical resin-made hollow needle according to claim 2 or 3,
wherein a distance between a distal end of the lateral hole and the proximal end of the distal end section is in a range of 1.8 to 5.0 mm.

5. The medical resin-made hollow needle according to any one of claims 1 to 4,
wherein the puncture end portion has a conical shape having a total length in a range of 2.1 to 4.0 mm, and a taper angle in a range of 14° to 25°.

6. The medical resin-made hollow needle according to any one of claims 1 to 5,
wherein the large-diameter section has a tapered shape that has an outer diameter increasing toward a proximal end thereof in a ratio in a range of 0.8% to 3.5%.

7. The medical resin-made hollow needle according to any one of claims 1 to 6, further comprising:
a rib that is provided on an outer surface of a proximal end portion of the hub section and is configured to be screwable with a threaded portion of the medical device; and
a protrusion that is provided on an outer surface of the hub section and extends in an axial direction.

8. A medical device set, comprising:
the medical resin-made hollow needle according to any one of claims 1 to 7; and
a medical device,
wherein the medical device is a pre-filled syringe that comprises an outer cylinder that includes an outer cylinder main body having a cylindrical shape and the Luer-taper nozzle provided at a distal end portion of the outer cylinder main body and including a distal end opening, a gasket that is slidably accommodated in the outer cylinder, a medical liquid that is filled in the outer cylinder, a sealing member that seals the distal end opening of the outer cylinder, and a plunger that is attached or attachable to a proximal end of the gasket.

9. The medical device set according to claim 8, further comprising:
the vial container that accommodates a drug configured to be dissolved or diluted with the medical liquid.

10. An outer cylinder with puncture portion, comprising:
an outer cylinder main body having a cylindrical shape; and
a hollow puncture portion that is provided in a distal end portion of the outer cylinder main body,
wherein the puncture portion comprises a distal end section that includes a puncture end portion at a distal end thereof, a large-diameter section having a diameter greater than a diameter of the distal end section and having a predetermined length, and a short tapered section that is provided between the large-diameter section and the distal end section,
wherein the distal end section includes a hole that communicates with an inside and an outside of the puncture portion, and the puncture end portion having a tapered shape,
wherein the distal end section has a total length in a range of 7 to 14 mm, and a maximum outer diameter in a range of 1.5 to 3.0 mm,
wherein the tapered section has a length in a range 0.3 to 4.7 mm, and a taper angle in a range of 10° to 50°,
wherein the large-diameter section has a length in a range 4 to 12 mm, and a distal end outer diameter that is greater than the maximum outer diameter of the distal end section by a range of 0.6 to 3.0 mm,
wherein the distal end section is configured to pierce an elastic container-sealing member of a vial container, and the distal end section and the tapered section are configured to pierce an elastic port-sealing member of a port of an infusion solution container, and
wherein the large-diameter section is configured to enter the elastic port-sealing member, and a state of the large-diameter section being entering into the elastic port-sealing member is configured to be maintained due to a pressure applied from the elastic port-sealing member.

11. The outer cylinder with puncture portion according to claim 10,
wherein the puncture end portion includes a solid tapered shape that has an outer diameter decreasing toward a distal end thereof, and the hole is a lateral hole that is provided proximal of the distal end of the distal end section by a predetermined length and is provided distal of a proximal end of the distal end section by a distance in a range of 1.8 to 5.0 mm.

12. The outer cylinder with puncture portion according to claim 11,
wherein a distance between a distal end of the lateral hole and the proximal end of the distal end section is in a range of 1.8 to 5.0 mm.

13. The outer cylinder with puncture portion according to any one of claims 10 to 12, further comprising:
a sealing cap that seals the hole.

14. A pre-filled syringe, comprising:
the outer cylinder with puncture portion according to any one of claims 10 to 13;
a gasket that is slidably accommodated in the outer cylinder;
a medical liquid that is filled in the outer cylinder; and
a plunger that is attached or attachable to a proximal end of the gasket.
